# EUROPEAN PATENT APPLICATION

(11) **EP 4 043 009 A1**
(43) Date of publication of application: **17.08.2022**
(21) Application number: 22156670.6
(22) Date of filing: 15.02.2022
(51) Int. Cl.: A61K 9/20, A61K 31/522, A61K 47/22, A61K 47/38, A61P 3/10

(54) **A PROCESS FOR FORMULATIONS OF LINAGLIPTIN OR A PHARMACEUTICALLY ACCEPTABLE SALT THEREOF**

(30) Priority: 15.02.2021 TR 202102097
(71) Applicant: SANOVEL ILAC SANAYI VE TICARET A.S., Istanbul 34460 (TR)
(72) Inventor: OZDEN, Aydan, 34460 Istanbul (TR); PEHLIVAN AKALIN, Nur, 34460 Istanbul (TR); SUNEL, Fatih, 34460 Istanbul (TR)
(74) Representative: Sevinç, Erkan

(57) **Abstract**

The present invention relates to a process for the preparation of a film coated tablet formulation comprising linagliptin or a pharmaceutically acceptable salt thereof. The process is simple, rapid, cost effective, time-saving and industrially convenient process and is created to eliminate the disadvantages of active ingredient.

## Description

### Field of the invention

The present invention relates to a process for the preparation of a film coated tablet formulation comprising linagliptin or a pharmaceutically acceptable salt thereof. The process is simple, rapid, cost effective, time-saving and industrially convenient process and is created to eliminate the disadvantages of active ingredient.

### Background of the invention

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

The chemical name of linagliptin is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

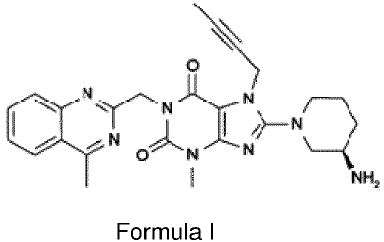

In general, DPP-4 inhibitors are not very stable compounds. Especially, in solid dosage forms, amine group containing DPP-4 inhibitors like linagliptin may react with many excipients or impurities of excipients. Therefore, it is as important in the process as the excipients used. Also, Linagliptin or a pharmaceutically acceptable salt thereof is used small proportion that can lead to considerable problems which are content uniformity, flowability and compressibility.

US patent publication 2011/0206766 A1 discloses a composition comprising a DPP-4 inhibitor and a partner drug and a basic agent for stabilizing said DPP-4 inhibitor against degradation.

WO2014/026939 patent application discloses a film coated tablet comprising a core and a coating, wherein the core is prepared by direct compression and comprises linagliptin or a pharmaceutically acceptable salt thereof, mannitol, copovidone, and magnesium stearate.

In the prior art, there are many pharmaceutical compositions comprising linagliptin or a pharmaceutically acceptable salt thereof, but it is needed to develop a composition which has a long-term stability for use and desired content uniformity, flowability and compressibility.

There is thus still a need for a process for the film coated tablet comprising linagliptin or a pharmaceutically acceptable salt thereof that provides a tablet having the desired stability, content uniformity, flowability and compressibility, in another words the disadvantages seen in the active substance will able to overcome.

In the present invention, the process is using standard techniques which is simple and cost-effective method.

### Detailed description of the Invention

The main object of the present invention is to provide an effective process for the preparation of a film coated tablet formulation comprising linagliptin or a pharmaceutically acceptable salt thereof which eliminate all aforesaid problems and bring additional advantages to the relevant prior art. The process provides the desired content uniformity, flowability and compressibility of the film coated tablet.

Linagliptin or a pharmaceutically acceptable salt thereof is used small proportion that can lead to considerable problems during the manufacture of the composition with regard to the uniformity of the content of active agent in the individual composition units.

It has surprisingly been found that a film coated tablet of excellent content uniformity, good flowability and showing improved dissolution can be obtained when the following steps are applied in wet granulation process, preferably fluid-bed spray granulation. Fluid-bed spray granulation efficiently counteracts the segregation of active agent, so it is observed that the desired stability, flowability, content uniformity and compressibility.

A process for the preparation of the film coated tablet comprising linagliptin or a pharmaceutically acceptable salt thereof comprising the following steps:
a) Mixing linagliptin or a pharmaceutically acceptable salt thereof, at least one disintegrant and at least one filler,
b) Dissolving at least binder in a solvent and obtained a granulation solution,
c) Granulating the granulation solution with the mixture at step (a) using spray granulation.

So, an easy method is created to eliminate the disadvantages of active ingredient and also the method is simple and cost-effective method. Also, this process helps to provide the desired dissolution profile.

In the process, linagliptin are put into the tank at the same time with at least one disintegrant and at least one filler, Thus, the desired mixture can be achieved without loss of active ingredient.

According to one embodiment, the amount of linagliptin or a pharmaceutically acceptable salt thereof is in the range of 1.0 to 10 %, preferably 1.0 to 8.0 %, more preferably it is 1.0 to 5.0 % by weight of total composition.

Suitable fillers are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment, microcrystalline cellulose is a filler which has the best flowability properties among the other fillers. In this invention, it further improves the disintegration of compositon as well as being a filler. In addition, it further enhances the compressibility by increasing the hardness of the tablet.

In this embodiment, the amount of microcrystalline cellulose is in the range of 70.0 to 90 %, preferably 76.0 to 88.0 %, more preferably it is 80.0 to 85.0 % by weight of total composition.

Suitable disintegrants are selected from the group comprising sodium starch glycolate, crosslinked polyvinil pyrrolidone (crospovidone), croscarmellose sodium, low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, alginic acid, alginates, sodium dodecyl sulphate, sodium glycine carbonate, sodium lauryl sulphate or mixtures thereof.

A sufficient amount of disintegrant that is neither too much nor too little to detrimentally alter the release of the active ingredient should be used to form solid oral dosage forms provided herein. However, disintegrants can be mixed with other excipient to increase effective disintegration of the tablet into smaller fragments.

In one embodiment of the invention, disintegrant is sodium starch glycolate, is also known as superdisintegrant, and the amount of sodium starch glycolate is in the range of 3.0% to 8.0% by weight in the composition and thus desired level of dissolution rate is provided.

Furthermore, combination of polyvinylpyrrolidone and sodium starch glycolate provides desired short disintegration time and desired dissolution properties in composition.

Improved content uniformity efficiently contributes to a marked increase in bioavailability. Improved content uniformity also favors to avoid toxicity in the otherwise possible event that the amount of drug substance would be too high. In the process, when a granulation solution is prepared with at least binder, then mixing with the mixture comprising active agents, it was observed that the desired content uniformity is provided. Especially, the problem caused by the use of small amounts of linagliptin has been prevented.

Suitable binders are selected from the group comprising polyvinylpyrrolidone, copovidone, hydroxypropylmethyl cellulose, hydroxypropyl cellulose, hydroxyethyl cellulose, mannitol, gelatin, pullulan, sodium alginate or mixtures thereof.

The present invention comprises one or more binders in an amount of from about 1% to about 20% by weight of the composition.

In one embodiment of the invention, binder is polyvinylpyrrolidone and the amount of polyvinylpyrrolidone is 1.0% and 5.0% by weight in the composition.

In general, DPP-4 inhibitors are not very stable compounds. Especially, in solid dosage forms, amine group containing DPP-4 inhibitors like linagliptin may react with many excipients or impurities of excipients. In this invention, it has been surprisingly found that using binder ensures high stability of linagliptin in a solid oral dosage composition. Especially polyvinylpyrrolidone is used as binder in the present invention. Polyvinylpyrrolidone has also disintegration property and it is used to enhance dissolution of poorly soluble linagliptin or a pharmaceutically acceptable salt thereof from solid-dosage forms.

According to an embodiment of the present invention, solvents are selected from the group comprising pure water, propylene glycol, glycerin, ethanol, polyethylene glycol or mixtures thereof.

According to an embodiment of the present invention, the solvent is pure water.

According to an embodiment of the present invention, spray granulation is used at step (c). The main objective of spray granulation is to improve the undesirable powder characteristics of raw materials (poor powder flow, fluffiness, segregation, etc.).

According to one embodiment of the present invention, the process for the preparation of the film coated tablet further comprises the following steps:
d) Drying the mixture, then sieving the mixture,
e) Adding at least one lubricant and mixing,
f) Compressing the mixture to form a tablet,
g) Coating tablets with film coating agents.

According to another embodiment, the lubricant is selected from the group comprising sodium stearyl fumarate, magnesium stearate, polyethylene glycol (PEG), sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably sodium stearyl fumarate.

According to one embodiment of the present invention, the process for the preparation of the film coated tablet comprises the following steps:
a) Mixing linagliptin or a pharmaceutically acceptable salt thereof, microcrystalline cellulose, sodium starch glycolate
b) Dissolving polyvinylpyrrolidone in pure water and obtained a granulation solution,
c) Granulating the granulation solution with the mixture at step (a) using spray granulation,
d) Drying the mixture, then sieving the mixture,
e) Adding sodium stearyl fumarate and mixing,
f) Compressing the mixture to form a tablet,
g) Coating tablets with film coating agents.

Coating may also preferably be used for moisture protection. Suitable coating agents are selected from the group comprising polymethacrylates, hydroxypropyl methylcellulose, lactose monohydrate, hydroxypropyl cellulose, polyvinyl alcohol (PVA), polyethylene glycol (PEG), talc, glycerine, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), all kinds of Opadry^{®}, pigments, dyes, titanium dioxide, macrogol, coloring agent or mixtures thereof.

Preferably, the film coated tablet of the present invention comprises linagliptin or a pharmaceutically acceptable salt thereof, polyvinylpyrrolidone, microcrystalline cellulose, sodium starch glycolate, sodium stearyl fumarate.

### Example 1: The film coated tablet comprising linagliptin

| | **(%) amount (w/w)** |
|---|---|
| Linagliptin | 1.0% - 10.0% |
| Microcrystalline cellulose | 70.0% - 90.0% |
| Polyvinylpyrrolidone | 1.0% - 5.0% |
| Sodium starch glycolate | 3.0% - 8.0% |
| Sodium stearyl fumarate | 0.1% - 5.0% |
| Coating agents | 0.5% - 8.0% |
| Total | 100 |

### Example 2: The film coated tablet comprising linagliptin

| | **(%) amount (w/w)** |
|---|---|
| Linagliptin | 2.7 |
| Microcrystalline cellulose | 82.1 |
| Polyvinylpyrrolidone | 2.9 |
| Sodium starch glycolate | 4.9 |
| Sodium stearyl fumarate | 2.0 |
| Coating agents | 5.4 |
| **Total** | **100** |

**Process for example 1 or 2**;
a) Mixing linagliptin, microcrystalline cellulose, sodium starch glycolate and charging the mixture to fluid bed granulator-dryer,
b) Adding polyvinylpyrrolidone in pure water and dissolving polyvinylpyrrolidone and then obtained granulation solution,
c) Then, granulating the mixture at step (a) with the granulation solution at step (b) using spray granulation,
d) Drying the mixture, then sieving the mixture,
e) Adding sodium stearyl fumarate and then mixing,
f) Then, pressing to form tablet
g) Coating tablets with coating agents.

## Claims

1. A process for the preparation of the film coated tablet comprising linagliptin or a pharmaceutically acceptable salt thereof comprising the following steps:
a) Mixing linagliptin or a pharmaceutically acceptable salt thereof, at least one disintegrant and at least one filler,
b) Dissolving at least binder in a solvent and obtained a granulation solution,
c) Granulating the granulation solution with the mixture at step (a) using spray granulation.

2. The process for the preparation of the film coated tablet according to claim 1, wherein the amount of linagliptin or a pharmaceutically acceptable salt thereof is in the range of 1.0 to 10 %, preferably 1.0 to 8.0 %, more preferably it is 1.0 to 5.0 % by weight of total composition.

3. The process for the preparation of the film coated tablet according to claim 1, wherein fillers are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

4. The process for the preparation of the film coated tablet according to claim 3, wherein filler is microcrystalline cellulose.

5. The process for the preparation of the film coated tablet according to claim 1, wherein disintegrants are selected from the group comprising sodium starch glycolate, crosslinked polyvinil pyrrolidone (crospovidone), croscarmellose sodium, low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, alginic acid, alginates, sodium dodecyl sulphate, sodium glycine carbonate, sodium lauryl sulphate or mixtures thereof.

6. The process for the preparation of the film coated tablet according to claim 5, wherein disintegrant is sodium starch glycolate.

7. The process for the preparation of the film coated tablet according to claim 1, wherein binders are selected from the group comprising polyvinylpyrrolidone, copovidone, hydroxypropylmethyl cellulose, hydroxypropyl cellulose, hydroxyethyl cellulose, mannitol, gelatin, pullulan, sodium alginate or mixtures thereof.

8. The process for the preparation of the film coated tablet according to claim 7, wherein binder is polyvinylpyrrolidone.

9. The process for the preparation of the film coated tablet according to claim 1, wherein solvents are selected from the group comprising pure water, propylene glycol, glycerin, ethanol, polyethylene glycol or mixtures thereof.

10. The process for the preparation of the film coated tablet according to claim 9, wherein the solvent is pure water.

11. The process for the preparation of the film coated tablet according to claim 1, wherein further comprises the following steps:
d) Drying the mixture, then sieving the mixture,
e) Adding at least one lubricant and mixing,
f) Compressing the mixture to form a tablet,
g) Coating tablets with film coating agents.

12. The process for the preparation of the film coated tablet according to claim 11, wherein the lubricant is selected from the group comprising sodium stearyl fumarate, magnesium stearate, polyethylene glycol (PEG), sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably sodium stearyl fumarate.

13. The process for the preparation of the film coated tablet according to claim 1, wherein comprises the following steps:
a) Mixing linagliptin, microcrystalline cellulose, sodium starch glycolate and charging the mixture to fluid bed granulator-dryer,
b) Dissolving polyvinylpyrrolidone in pure water and obtained a granulation solution,
c) Granulating the granulation solution with the mixture at step (a) using spray granulation,
d) Drying the mixture, then sieving the mixture,
e) Adding sodium stearyl fumarate and mixing,
f) Compressing the mixture to form a tablet,
g) Coating tablets with film coating agents.
